# EUROPÄISCHE PATENTANMELDUNG

(11) **EP 3 598 120 A1**
(43) Veröffentlichungstag der Anmeldung: **22.01.2020**
(21) Anmeldenummer: 19187003.9
(22) Anmeldetag: 18.07.2019
(51) Int. Cl.: G01N 27/62, G01N 33/00

(54) **SENSORVORRICHTUNG UND EIN VERFAHREN ZUR ERFASSUNG EINES KOHLENWASSERSTOFF-ANTEILS**

(30) Priorität: 19.07.2018 DE 102018212089
(71) Anmelder: Hochschule Karlsruhe, 76133 Karlsruhe (DE)
(72) Erfinder: Kettner, Maurice, 76199 Karlsruhe (DE); Scholl, Fino, 90403 Nürnberg (DE)
(74) Vertreter: Altmann Stößel Dick Patentanwälte PartG mbB

(57) **Zusammenfassung**

Es wird eine Sensorvorrichtung (110) zur Erfassung mindestens eines Kohlenwasserstoff-Anteils in mindestens einem Gas vorgeschlagen. Die Sensorvorrichtung (110) umfasst eine für das Gas zugängliche Messzelle (116) mit mindestens einem dem Gas zugänglichen Heizelement (114). Weiterhin umfasst die Sensorvorrichtung (110) mindestens eine Elektrode (130), insbesondere ein Elektrodenpaar, zur Erfassung mindestens eines Ionenstroms in dem Gas.

## Beschreibung

### Technisches Gebiet

Die Erfindung betrifft eine Sensorvorrichtung und ein Verfahren zur Erfassung mindestens eines Kohlenwasserstoff-Anteils in mindestens einem Gas. Die Sensorvorrichtung und das Verfahren können insbesondere eingesetzt werden zur Emissionskontrolle von Fahrzeugen, in Kraftwerksantrieben wie beispielsweise Blockheizkraftwerken oder Biogasanlagen, sowie in Schiffsantrieben oder auch in Deponieanlagen. Auch andere Anwendungen, bei denen ein Kohlenwasserstoff-Anteil in Gasen ermittelt werden muss, sind möglich.

### Technischer Hintergrund

In zahlreichen technischen Anwendungsgebieten ist eine Erfassung von Kohlenwasserstoff-Anteilen in Gasen erforderlich. Bei diesen Anwendungen kann es sich einerseits um die Überwachung von Anlagen handeln, bei denen Gase mit Kohlenwasserstoff-Anteilen entstehen, wie beispielsweise Biogasanlagen, Deponieanlagen oder Tankentlüftungssystemen. Andererseits kann es sich jedoch auch um Anwendungen handeln, bei denen Kohlenwasserstoff-Gasgemische für Verbrennungen eingesetzt werden, beispielsweise die Kraftwerkstechnik, Schiffsantriebe oder auch die Kraftfahrzeugtechnik.

So ist beispielsweise die Forderung eines nachhaltigen Individualverkehrs ein wesentlicher Treiber für die Entwicklungen der Automobilindustrie. Neben der Steigerung der Energieeffizienz steht die Reduktion der Schadstoffemissionen im Fokus der Hersteller. Die getroffenen verbrauchs- und verbrennungstemperatursenkenden Maßnahmen waren überwiegend auf die CO₂- und die NOₓ-Reduktion gerichtet. Mit der Einführung der Euro-6d-temp-Gesetzgebung rückt das Gefährdungspotential der Partikelemissionen sowie der Gruppe der unverbrannten Kohlenwasserstoffe (HC) ergänzend in den Vordergrund. Ebenso wird mit Inkrafttreten die Messung von Abgasemissionen in der Realfahrt (RDE: Real Driving Emissions) ab 2017 vorgeschrieben. Die HC-Emissionen wurden jedoch vorerst trotz des hohen ökotoxikologischen Gefährdungspotentials insbesondere aufgrund fehlender Messtechnik, die den Anforderungen an Sicherheit, Genauigkeit und zeitlicher Auflösung genügt, vom Gesetzgeber bei der Realfahrtmessung in Europa ausgeschlossen. Gegenwärtig begünstigen Fahr-Strategien wie der Start-Stopp-, Segel- und der Hybrid-Betrieb das betriebsbedingte Auskühlen der Abgasnachbehandlungssysteme und erschweren, insbesondere bei erdgasbetriebenen Fahrzeugen, die Oxidation von HC-Emissionen. Darüber hinaus bestätigen Untersuchungen an Hybridfahrzeugen, dass zwar eine signifikante Reduzierung der NOₓ-Emissionen erzielt wurde, dementgegen aber ein Anstieg der CO- und HC-Emissionen im realen Fahrbetrieb resultiert. Ferner wird intensiv an Dual-Fuel Konzepten bei Nutzfahrzeugen und Schiffsantrieben entwickelt, die kombiniert mit einem Ottomotorkraftstoff (beispielsweise Erdgas, Flüssiggas oder Ethanol) und einem Dieselmotorkraftstoff (beispielsweise Diesel, Biodiesel oder Pflanzenöl) betrieben werden. Konzeptbedingt tritt jedoch im Dual-Fuel Betrieb angesichts der äußeren Gemischbildung in der Ventilüberschneidungsphase und des mageren Ausbrands Methanschlupf auf, welcher in einem gegenläufigen Verhältnis zu anderen Optimierungsparametern, insbesondere auch dem HC-Ausstoß steht. Gelangen beispielsweise unverbrannte Kohlenwasserstoffe in die Abgasanlage, werden diese entweder in die Umwelt emittiert oder (bei ausreichendem Restsauerstoffgehalt im Abgas) wird bei deren Umsetzung in kurzer Zeit so viel Wärme freigesetzt, dass der Alterungsprozess des Katalysators durch thermische Überlastung beschleunigt wird. Die ökotoxikologische Wirkung von HC-Emissionen sowie das hohe Treibhauspotential von Methan-HC erfordern ihre Quantifizierung im realen Fahrbetrieb in Echtzeit.

Aktuelle Messsysteme für HC-Emissionen wie ein Flammenionisationsdetektors (FID), welcher auf der Leitfähigkeit einer Knallgasflamme unter der Beimischung des zu messenden Gases zwischen zwei Elektroden beruht, sind in der Regel aus Sicherheitsaspekten Prinzip bedingt nachteilig, beispielsweise aufgrund einer notwendigen Mitnahme von hochentzündlichem Wasserstoff als Trägergas im Fahrzeug.

Andere Messprinzipien weisen in vielen Fällen Messfrequenzen auf, welche ein erhebliches Verbesserungspotenzial beinhalten, so beispielsweise potentiometrische Mischpotentialsensoren. Auch DE 10 2006 033 110 A1 beschreibt einen Sensor zur Messung eines Gehaltes von Kohlenwasserstoffen HC in einer Gasatmosphäre, insbesondere eines Abgases einer Verbrennungskraftmaschine. Der Sensor umfasst ein Messelement, das eine temperaturabhängige Eigenschaft aufweist und eine katalytische Beschichtung zur chemischen Umsetzung von HC trägt. Der Sensor umfasst weiterhin ein Referenzelement ohne katalytische Beschichtung, das ebenfalls eine temperaturabhängige elektrische Eigenschaft aufweist, sowie Mittel zur Messung der temperaturabhängigen Eigenschaft des Messelements und des Referenzelements und zur Bestimmung des HC-Gehaltes in Abhängigkeit eines Vergleichs der temperaturabhängigen Eigenschaft des Messelements und der elektrischen Eigenschaft des Referenzelements. Das Messelement und das Referenzelement können insbesondere jeweils ein Thermoelement oder ein Widerstandsthermometer sein. Insgesamt wäre bei Kohlenwasserstoff-Messungen, insbesondere für Kraftfahrzeuganwendungen, eine höhere Messfrequenz wünschenswert.

In DE 197 57 112 A1 wird ein Gassensor zur Messung eines Sauerstoff- und/oder Luft/Kraftstoff-Lambda-Verhältnisses und von Stickoxiden in Gasgemischen beschrieben. Um eine Anzahl von gasförmigen Bestandteilen zuverlässig zu messen, ist der Sensor mit einer Referenzelektrode, die einen konstanten Sauerstoffpartialdruck darstellt, und mindestens zwei Elektrodenpaaren versehen, wobei diese Elektrodenpaare jeweils einer gemeinsamen Elektrode zugeordnet sind. Die Erfindung umfasst auch Festelektrolyte, die direkt auf den Elektroden zwischen beiden Elektroden eines Elektrodenpaares angeordnet sind, wobei der zwischen einem ersten Elektrodenpaar befindliche Festelektrolyt gasdurchlässig und der zwischen einem zweiten Elektrodenpaar befindliche Festelektrolyt gasdicht ist. Das zweite Elektrodenpaar dient zur potentiometrischen oder amperometrischen Messung der Stickoxidkonzentration. Das erste Elektrodenpaar ist zum Anlegen eines Stroms oder einer Spannung an Sauerstoffpumpen konfiguriert. Die gemeinsame Elektrode bildet die Kathode des ersten Elektrodenpaares und die zweite Elektrode des zweiten Elektrodenpaares ist als Referenzelektrode auf der Referenzgasseite des Festelektrolyten des zweiten Elektrodenpaares ausgebildet.

In CN 1 527 051 A wird ein Verfahren und ein Sensor zum Detektieren von organischem Gas mit Nanomaterial beschrieben. Basierend auf dem Prinzip des Nanomaterials mit hoher katalytischer Aktivität zur Erzeugung von organischen Molekülionen und dem Merkmal, dass die elektrochemische Modifikation von empfindlichem Material keinen Einfluss auf die Ionenmessung hat, bestimmt das Verfahren die Konzentration von organischem Gas durch Erfassen des Ionenstroms, welcher bei der katalytischen Reaktion auf Nanomaterialoberflächen entsteht und dazu gebracht wird, unter einem elektrischen Feld in eine bestimmte Richtung einzuwandern. Der Sensor weist eine Struktur auf, die ein Keramiksubstrat, eine elektrische Heizung, eine Elektrode mit einem katalytischen Nanofilm und die gegenüberliegende Elektrode umfasst.

In JP S61-265 562 A wird ein gasförmiges Gemisch aus Probengas und Hilfsbrenngas mit einem Verbrennungskatalysatorelement in Kontakt gebracht, um dasselbe zu erhitzen und das Gemisch anschließend in einem Ofen zu verbrennen. Ziel ist es, die Unregelmäßigkeit in der Empfindlichkeit des Kohlenwasserstoffs zu verringern und ein Wasserstoff-Flammenion mit hoher Genauigkeit zu erfassen.

In JP 2000 162 187 A wird ein Kohlenwasserstoff-Analysegerät mit einer einfachen und kostengünstigen Struktur bereitgestellt, welches in der Lage ist, ein vorbehandeltes Probengas effektiv zu verwenden und die plötzliche Änderung des Hintergrundwerts zum Zeitpunkt der Kalibrierung zu verhindern. Das Gerät umfasst einen Wasserstoff-Flammenionisationsdetektor und einen Gaszufuhrkanal zum abwechselnden Zuführen eines Probengases und eines luftbasierten Kalibrierungsgases, einen Zufuhrkanal für Brenngas und einen Zufuhrkanal zum Zuführen von Gas, welcher mit einer Nullgasreinigungseinrichtung an den Wasserstoffionisationsdetektor angeschlossen ist. Der Gaszufuhrkanal ist mit einer Gasumschalteinheit versehen, die einen Ausstoßteil für überschüssiges Gas aufweist. Der Zufuhrkanal zum Stützen von dem Gas ist mit dem Ausstoßteil für überschüssiges Gas verbunden, um das überschüssige Gas der Wasserstoffflammenionisation zuzuführen.

In DE 30 05 928 A1 wird ein Geber zur Aufnahme von Ionenströmen in Kohlenwasserstoffgemischen beschrieben. Für hohe Temperaturen sind in an sich bekannter Weise auf einem isolierten Träger hoher Temperaturfestigkeit ein Heizleiter und mindestens zwei Messelektroden aufgebracht.

In DE 10 2006 033 110 A1 wird ein Sensor zur Messung eines Gehaltes von Kohlenwasserstoffen HC in einer Gasatmosphäre, insbesondere eines Abgases einer Verbrennungskraftmaschine beschrieben. Der Sensor umfasst ein Messelement, das eine temperaturabhängige Eigenschaft aufweist und eine katalytische Beschichtung zur chemischen Umsetzung von HC trägt. Weiterhin umfasst der Sensor ein Referenzelement ohne katalytische Beschichtung, das ebenfalls eine temperaturabhängige elektrische Eigenschaft aufweist, ein Mittel zur Messung der temperaturabhängigen Eigenschaft des Messelements und des Referenzelements und zur Bestimmung des HC-Gehaltes in Abhängigkeit eines Vergleichs der temperaturabhängigen Eigenschaft des Messelements und der elektrischen Eigenschaft des Referenzelements.

In DE 195 02 285 A1 beschreibt einen flammenloser Ionisationsdetektor (FLID), der unabhängig von Brenngasen betrieben werden kann, für den Einsatz in mobilen Analysengeräten. Zur flammenlosen Ionisation wird ein elektrisch beheizbares Ionisationsröhrchen aus hochreiner Aluminiumoxidkeramik eingesetzt. Die Heizung ist in Form einer Platinspirale um das Ionisationsröhrchen angebracht und mit einer Schicht keramischen Materials überzogen. Die typische Arbeitstemperatur des Ionisationsröhrchens beträgt heizstromabhängig 600 °C bis 1200°C. Der FLID ermöglicht den einfachen Aufbau von mobilen Analysengeräten wie sie z. B. im Bereich der Produkt- und produktionsbegleitenden sowie der Umweltanalytik eingesetzt werden. Der Betrieb erfolgt in Verbindung mit einem chromatographischen Trennsystem als unspezifischer Sensor für ionisierbare Substanzen in Gasen z. B. für die Überwachung von Räumen, Kanälen und technischen Produktionsanlagen. Basierend auf dem Messprinzip werden auch Substanzen nachgewiesen (z. B. Schwefel- und Stickoxide), für die der FID keine bzw. nur eine geringe Empfindlichkeit verfügt.

Somit besteht insgesamt ein Bedarf an Kohlenwasserstoff-Sensoren zur Messung der Konzentration von Kohlenwasserstoffen in einem Gas, welche einerseits strengen Sicherheitsanforderungen genügen und beispielsweise die Mitführung von entzündlichen Trägergasen wie beispielsweise Wasserstoff vermeiden. Zudem wäre, im Vergleich zu bekannten Kohlenwasserstoff-Sensoren, eine Verringerung der Wartungsintensität sowie eine Verringerung der Häufigkeit einer Kalibrierung wünschenswert. Für den Einsatz in Kraftfahrzeugen wäre allgemein auch eine Eignung für den Einsatz im fahrenden Fahrzeug wünschenswert

### Aufgabe der Erfindung

Es wäre daher wünschenswert, eine Sensorvorrichtung und ein Verfahren zur Erfassung eines Kohlenwasserstoff-Anteils in mindestens einem Gas bereitzustellen, welche die Nachteile bekannter Vorrichtungen und Verfahren der genannten Art zumindest weitgehend vermeiden. Insbesondere sollte die Sensorvorrichtung robust ausgestaltet sein, geeignet auch für den Einsatz in einem fahrenden Fahrzeug sein und sollte eine geringe Wartungsintensität aufweisen.

### Offenbarung der Erfindung

Diese Aufgabe wird adressiert durch eine Sensorvorrichtung und ein Verfahren zur Erfassung eines Kohlenwasserstoff-Anteils in mindestens einem Gas, mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf', "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, das das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

In einem ersten Aspekt wird daher eine Sensorvorrichtung zur Erfassung mindestens eines Kohlenwasserstoff-Anteils in mindestens einem Gas vorgeschlagen. Der Begriff "Sensorvorrichtung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche mindestens eine Eigenschaft einer Umgebung, eines Stoffs, eines Systems oder eines Elements erfassen und in mindestens eine Messgröße umwandeln kann. Insbesondere kann die Sensorvorrichtung eingerichtet sein, um mindestens eine Eigenschaft mindestens eines gasförmigen Mediums zu erfassen, beispielsweise mindestens eine chemische und/oder physikalische Eigenschaft. Unter einer Erfassung kann dabei allgemein eine qualitative oder insbesondere quantitative Erfassung der mindestens einen Eigenschaft verstanden werden, insbesondere eine Messung.

Der Begriff "Kohlenwasserstoff-Anteil", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf mindestens eine den Anteil von Kohlenwasserstoffen in dem Gas beschreibende Messgröße beziehen, insbesondere ausgewählt aus der Gruppe bestehend aus: einem Partialdruck von Kohlenwasserstoff-Gasen in dem Gas; einer Konzentration von Kohlenwasserstoffen in dem Gas; einer Absolutmenge von Kohlenwasserstoffen in dem Gas; einem Volumenprozent-Anteil von Kohlenwasserstoffen in dem Gas; einem Massenprozent-Anteil von Kohlenwasserstoffen in dem Gas.

Der Begriff "Kohlenwasserstoffe", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf chemische Verbindungen beziehen, welche Kohlenstoff und Wasserstoff aufweisen. Dabei können die chemischen Verbindungen ausschließlich aus Kohlenstoff und Wasserstoff bestehen oder können auch weitere Elemente umfassen, beispielsweise Substituenten der Wasserstoffatome. Die Kohlenwasserstoffe können insbesondere gasförmig sein. Die Kohlenwasserstoffe können insbesondere Methan umfassen. Auch andere Kohlenwasserstoffe sind jedoch grundsätzlich einsetzbar und erfassbar. Dabei kann die Sensorvorrichtung eingerichtet sein, um den Kohlenwasserstoff-Anteil summarisch über alle Kohlenwasserstoffe hinweg oder über mehrere Kohlenwasserstoffe hinweg zu erfassen, oder kann alternativ auch eingerichtet sein, um selektive den Kohlenwasserstoff-Anteil einer oder mehrerer spezieller KohlenwasserstoffVerbindungen zu erfassen.

Der Begriff "Gas", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein grundsätzlich beliebiges gasförmiges Element, eine grundsätzlich beliebige gasförmige Verbindung oder auch um ein grundsätzlich beliebiges Gemisch gasförmiger Elemente und/oder Verbindungen beziehen. Insbesondere kann das Gas mindestens ein gasförmiges Element oder mindestens eine gasförmige Verbindung umfassen, ausgewählt aus der Gruppe bestehend aus: Luft; Stickstoff; Sauerstoff; einem Trägergas; Kohlenwasserstoffen in einem Trägergas, insbesondere einem Trägergas mit einem oder mehreren Gasen ausgewählt aus der Gruppe bestehend aus Luft, Stickstoff, Sauerstoff, Wasserdampf, Kohlendioxid, Stickoxiden oder Schwefeldioxid.

Die Sensorvorrichtung umfasst mindestens eine für das Gas zugängliche Messzelle mit mindestens einem dem Gas zugänglichen Heizelement sowie optional mindestens einem dem Gas zugänglichen Katalysatorelement zur Erzeugung von Ionen in dem Gas. Das Heizelement kann zumindest teilweise als in die Messzelle hineinragender Heizstab ausgestaltet sein.

Der Begriff "Messzelle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung oder ein System beziehen, welches mindestens einen vollständig oder teilweise umschlossenen Innenraum aufweist, in welchem eine Messung der mindestens einen Eigenschaft erfolgt. Der Begriff "für das Gas zugänglich" kann im vorliegenden Zusammenhang insbesondere bedeuten, dass die Messzelle mindestens eine Öffnung und/oder mindestens einen Zugang aufweist, durch welche bzw. welchen das mindestens eine Gas in den Innenraum eintreten kann. Dabei kann das Gas den Innenraum durchströmen oder auch lediglich unbewegt in dem Innenraum vorliegen.

Der Begriff "Heizelement", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um mindestens eine weitere Vorrichtung, mindestens ein Element, oder mindestens einen Stoff oder ein Stoffgemisch zu erwärmen. Insbesondere kann das Heizelement mindestens ein elektrisches Heizelement sein, beispielsweise mindestens ein elektrisches Heizelement mit mindestens einem Heizwiderstand. Das Heizelement kann zumindest teilweise als Heizstab ausgestaltet sein, also als stabförmiges Heizelement. Das Heizelement kann weiterhin als eine Heizoberfläche ausgestaltet sein. Die Heizoberfläche kann insbesondere als elektrisch regelbare Heizoberfläche ausgestaltet sein. Der Begriff "Heizstab" bezeichnet grundsätzlich ein beliebiges Element, welches eingerichtet ist, in geeigneter Weise elektrische Energie in innere Energie umzuwandeln. Dies kann beispielsweise nach dem ohmschen Prinzip (elektrischer Widerstand) oder beispielsweise durch eine induktive Beheizung erfolgen. Basiert die Umwandlung nach dem ohmschen Prinzip, so kann der Heizstab als ohmscher Heizstab bezeichnet werden. Für eine Verwendung in der Sensorvorrichtung gemäß der vorliegenden Erfindung bieten sich insbesondere Heizelemente an, bei denen aus Betriebsgrößen des Heizelements eine Temperatur bestimmt werden kann, insbesondere damit eine Temperaturregelung realisiert werden kann. Im Falle des ohmschen Heizstabs kann dies insbesondere derart erfolgen, dass man eine Temperaturabhängigkeit eines elektrischen Widerstands für die Temperaturregelung nutzt. Hierzu kann im Betrieb eine Versorgungsspannung sowie ein Versorgungsstrom gemessen werden, um den elektrischen Widerstand zu bestimmen.

Das Heizelement kann eingerichtet sein, auf eine Temperatur von mindestens 1000 °C, vorzugsweise von mindestens 1100 °C, vorzugsweise von mindestens 1200 °C, vorzugsweise von mindestens 1300 °C, vorzugsweise von mindestens 1400 °C, vorzugsweise von mindestens 1500 °C, vorzugsweise von mindestens 1600 °C, vorzugsweise von mindestens 1700 °C, vorzugsweise von mindestens 1800 °C, vorzugsweise von mindestens 1900 °C, vorzugsweise von mindestens 2000 °C, erhitzt zu werden. Insbesondere kann die Steuerung, welche im Folgenden noch näher beschrieben wird, eingerichtet sein, das Heizelement auf die Temperatur zu erhitzen. Das Heizelement kann mindestens eine Oberfläche aufweisen. Bei der Temperatur kann es sich insbesondere um eine Oberflächentemperatur einer Oberfläche des Heizelements handeln. Somit kann durch ausreichend hohe Temperaturen das Gas direkt ionisiert werden und es kann somit vollständig auf einen Einsatz eines Reaktivgases verzichtet werden.

Insbesondere kann das Heizelement mindestens eine Glühkerze umfassen. Eine Glühkerze kann dabei insbesondere ein Heizelement für den Einsatz in Kraftfahrzeugen sein, bei welchem ein in der Regel stabförmiges Heizelement, bei älteren Systemen auch beispielsweise als wendelartig geformter Widerstanddraht ausgebildetes Heizelement, aus einem Glühkerzengehäuse herausragt, wobei das Glühkerzengehäuse beispielsweise derart in eine Wand eingesteckt oder eingeschraubt werden kann, dass das stabförmige Heizelement in eine von der Wand umschlossene Kammer oder einen Raum hineinragen kann. Die Glühkerze kann standardmäßig in Dieselmotoren als Kaltstarthilfe verwendet werden. Die Glühkerze kann aus mindestens einem metallischen Material und/oder aus einem zumindest teilweise elektrisch leitfähigen keramischen Werkstoff hergestellt sein.

Das Heizelement kann insbesondere mindestens eine Oberfläche aufweisen, welche eingerichtet ist, Wärmeenergie zugeführt zu bekommen. Die Oberfläche kann eingerichtet sein, die Wärmeenergie an eine Umgebung des Heizelements abzugeben. Das Heizelement kann ein flammenfreies Heizelement sein. Das Heizelement kann eingerichtet sein, das Gas zu beheizen, insbesondere für eine Ionisierung des Gases.

Der Begriff "dem Gas zugänglich" ist im vorliegenden Zusammenhang analog zu dem Begriff "für das Gas zugänglich" gemäß seiner üblichen und gängigen Bedeutung zu verstehen, nach dem Verständnis des Fachmanns. Insbesondere kann der Begriff im Zusammenhang mit dem Heizelement bedeuten, dass mindestens eine Oberfläche des Heizelements, insbesondere mindestens eine beheizbare Oberfläche, mit dem Gas derart in Kontakt geraten kann, dass ein Wärmeübertrag von dem Heizelement auf das Gas erfolgen kann. Beispielsweise kann das Heizelement in den Innenraum der Messzelle hineinragen, derart, dass mindestens eine Oberfläche des Heizelements, beispielsweise des stabförmigen Heizelements und insbesondere der Glühkerze, mit dem Gas in dem Innenraum direkt oder indirekt in Berührung gelangt, so dass ein Wärmeübertrag von dem Heizelement an das Gas erfolgen kann, wodurch eine Ionisierung des Messgases eingeleitet wird.

Die Sensorvorrichtung kann, wie oben ausgeführt, mindestens ein dem Gas zugängliches Katalysatorelement zur Beeinflussung der Ionenbildung in dem Gas aufweisen. Der Begriff "Beeinflussung der Ionenbildung in dem Gas" kann insbesondere ein Erzeugen von Ionen in dem Gas und/oder eine Unterstützung der Ionenbildung in dem Gas umfassen. Dieses optionale Katalysatorelement kann getrennt von dem Heizstab ausgebildet sein, kann jedoch auch ganz oder teilweise mit dem oder in den Heizstab integriert sein, beispielsweise in Form einer katalytischen Beschichtung des Heizstabes.

Allgemein kann die Ionenbildung temperaturabhängig sein und von einer Gaszusammensetzung abhängen. Beispielsweise kann eine Ionenstrommessung in Brennräumen eine starke Abhängigkeit von einer Verbrennungstemperatur zeigen, die beispielsweise über ein Verbrennungsluftverhältnis variiert werden kann. Daher kann es grundsätzlich mittels der Sensorvorrichtung gemäß der vorliegenden Erfindung möglich sein, eine Ionenzahl durch eine Veränderung der Temperatur des Heizelements einzustellen. Darüber hinaus kann eine Probengastemperatur in der Sensorvorrichtung eingestellt bzw. verändert werden. Dies kann einen Einfluss auf eine Empfindlichkeit der Sensorvorrichtung haben.

Der Begriff "Katalysatorelement", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung oder ein Bauteil beziehen, welches bei Kontakt mit einem Stoff oder Stoffgemisch eine katalytische Reaktion in dem Stoff oder Stoffgemisch auslösen oder begünstigen kann. Insbesondere kann das Katalysatorelement ein festes Bauteil sein, welches mindestens eine katalytisch wirksame Oberfläche aufweist, welche mit dem Gas in der Messzelle in Kontakt gerät und eine heterogene Katalyse in dem Gas bewirkt. Analog zu dem Heizelement ist der Begriff "dem Gas zugänglich" in diesem Zusammenhang wiederum derart zu verstehen, wie es dem Verständnis des Fachmanns entspricht. Insbesondere kann sich der Begriff dahingehend verstehen lassen, dass das Katalysatorelement mindestens eine Katalysatoroberfläche aufweist, welche direkt oder indirekt mit dem Gas in Verbindung gerät, so dass eine katalytische Reaktion durch das Katalysatorelement in dem Gas bewirkt oder begünstigt werden kann. Diese Katalysatoroberfläche kann beispielsweise eine glatte oder auch eine poröse Oberfläche sein. Das Katalysatorelement kann beispielsweise in den Innenraum der Messzelle hineinragen oder kann, wie unten noch näher beschrieben wird, insbesondere auch den Innenraum ganz oder teilweise umschließen oder beispielsweise auch mindestens eine Wand oder mindestens einen Wandabschnitt des Innenraums bilden.

Das optionale Katalysatorelement ist derart eingerichtet, dass dieses katalytisch eine Ionenbildung in dem Gas bewirkt oder begünstigt, insbesondere die Bildung von Ionen aus Kohlenwasserstoffinolekülen. Dementsprechend ist die durch das Katalysatorelement katalysierte Reaktion im vorliegenden Fall die Bildung von Ionen in dem Gas, insbesondere Kohlenwasserstoff-Ionen.

Das Katalysatorelement kann insbesondere mindestens ein katalytisch wirksames Element aufweisen, insbesondere mindestens ein katalytisch wirksames Element ausgewählt aus der Gruppe bestehend aus: Platin, Palladium, Rhodium. Auch Kombinationen der genannten Elemente sind möglich. Insbesondere kann das Katalysatorelement ganz oder teilweise als Keramik ausgebildet sein, wobei beispielsweise Partikel des mindestens einen katalytisch wirksamen Elements, beispielsweise Platin und/oder Palladium und/oder mindestens eine Legierung aus zwei oder mehr katalytisch wirksamen Elementen, in die Keramik eingebettet sind oder anderweitig Bestandteil der Keramik sind. Beispielsweise kann die Keramik ganz oder teilweise glatt ausgebildet sein, so dass die katalytisch wirksamen Partikel an mindestens einer Oberfläche der Keramik für das Gas zugänglich sind. Alternativ oder zusätzlich kann die Keramik jedoch auch ganz oder teilweise porös ausgestaltet sein, wobei beispielsweise das Gas in Poren der Keramik eindringen kann, um dort mit den katalytisch wirksamen Partikeln in Kontakt zu geraten. Wiederum alternativ oder zusätzlich kann das Katalysatorelement auch eine mit dem mindestens einen katalytisch wirksamen Element beschichtete Oberfläche aufweisen, beispielsweise mindestens eine Beschichtung mindestens einer keramischen Oberfläche und/oder mindestens eine Beschichtung mindestens einer metallischen Oberflächen, beispielsweise einer Oberfläche einer Stahl-Glühkerze. Auch andere Ausgestaltungen sind denkbar.

Die Sensorvorrichtung umfasst weiterhin mindestens eine Elektrode zur Erfassung eines Ionenstroms in dem Gas. Der Begriff " Elektrode", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung oder ein Element mit elektrisch leitenden Eigenschaften beziehen, beispielsweise ein metallisches Element, welches mit mittels mindestens einer Zuleitung elektrisch kontaktierbar ist, so dass dieses Element beispielsweise mit mindestens einer elektrischen Vorrichtung verbindbar ist, insbesondere mindestens einer Vorrichtung ausgewählt aus der Gruppe bestehend aus einer Stromquelle, einer Spannungsquelle, einer Strommessvorrichtung, einer Spannungsmessvorrichtung. Dabei kann die Elektrode dem Gas direkt zugänglich sein, beispielsweise indem die Elektrode mindestens eine elektrisch leitfähige Oberfläche aufweist, welche mit dem Gas in Kontakt gerät. Diese Oberfläche kann beispielsweise eben oder auch gekrümmt ausgestaltet sein. Es können beispielsweise eine einzige Elektrode, zwei Elektroden oder auch mehr als zwei Elektroden vorgesehen sein, beispielsweise Elektroden mit unterschiedlichem Potenzial und/oder unterschiedlicher Spannung, so dass in dem Gas ein Ionenstrom hin zu mindestens einer Elektrode erzeugt wird. Die Sensorvorrichtung kann daher mindestens ein Elektrodenpaar aufweisen. Beispielsweise können mindestens zwei gegensätzlich oder unterschiedlich geladene Elektroden in den Innenraum der Messzelle hineinragen, einen Bestandteil einer Wand des Innenraums bilden oder in anderer Weise für das Gas in dem Innenraum zugänglich angeordnet sein. Insbesondere kann die mindestens eine Elektrode in dem Innenraum der Messzelle angeordnet sein. Die eine oder mehrere Elektroden können beispielsweise über eine oder mehrere Zuleitungen mit einem Elektrodenpotenzial beaufschlagbar sein, so dass beispielsweise über die Zuleitung der Ionenstrom in dem Gas initiiert werden kann und auch als elektrischer Strom durch die Zuleitung gemessen werden kann. Dabei kann mindestens eine Elektrode auch ganz oder teilweise mit mindestens einem anderen Element der Sensorvorrichtung zusammengefasst sein, beispielsweise mit dem Heizelement, wobei bei einer derartigen Zusammenfassung vorzugsweise mindestens eine weitere Gegenelektrode vorgesehen ist, so dass ein Ionenstrom zwischen der mit einem anderen Element zusammengefassten Elektrode und der Gegenelektrode erfasst werden kann.

Der Begriff "Ionenstrom", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Bewegung von Ionen, also geladener Moleküle oder Atome, beziehen, insbesondere in dem Gas. Der Ionenstrom kann beispielsweise zwischen mindestens zwei Elektroden oder zwischen einer Elektrode und einer Gegenelektrode, erfolgen. Zur Erfassung des Ionenstroms kann beispielsweise ein elektrischer Strom durch mindestens eine Zuleitung zu der mindestens einen Elektrode erfasst werden, beispielsweise mittels mindestens eines Strommessgeräts, beispielsweise eine Strommessung über mindestens einen Messwiderstand. Auch andere elektrische Möglichkeiten zur Erfassung des Ionenstroms sind jedoch grundsätzlich einsetzbar.

Aus Molekülen können bei hohen Temperaturen, insbesondere durch thermische und/oder chemische Reaktionen, insbesondere bei Überschreitung einer Ionisierungsenergie bzw. einer Aktivierungsenergie, beispielweise bei einer Oxidation, Ionen entstehen. Daher liegen beispielsweise in Flammen üblicherweise mehr Ionen vor als beispielsweise in einer Umgebungsluft. Beispielsweise können Gasheizungen in Kombination mit einem Ionenstromsensor für eine Überwachung einer Flamme im Einsatz sein.

Im Rahmen der vorliegenden Erfindung können Ionen grundsätzlich durch eine Beheizung des Gases erzeugt werden. Insbesondere können die Ionen durch eine elektrisch beheizbare heiße Oberfläche in der Sensorvorrichtung erzeugt werden. Die elektrisch beheizbare heiße Oberfläche kann beispielsweise eine Oberfläche des Heizelements sein. Insbesondere kann das Heizelement, wie oben ausgeführt, der Heizstab, die Glühkerze oder der Glühstift sein. Das Heizelement, insbesondere der Glühstift, selbst kann auch Bestandteil des Ionenstromsensors sein, da das Heizelement, insbesondere der Glühstift, eine der Messelektroden darstellen kann.

Das optionale Katalysatorelement kann insbesondere derart ausgestaltet sein, dass das Katalysatorelement das Heizelement zumindest teilweise ringförmig umschließt. So kann beispielsweise das Heizelement ganz oder abschnittsweise stabförmig ausgebildet sein und kann beispielsweise axial auf einer Achse der Messzelle angeordnet sein. Das Katalysatorelement kann dann beispielsweise ganz oder abschnittsweise als Hohlzylinder ausgebildet sein, beispielsweise mit einem durchgehenden Innenraum oder auch mit einem in Segmente eingeteilten Innenraum, und kann beispielsweise das Heizelement in mindestens einem Abschnitt konzentrisch umschließen. Auch eine andere Art der Ausgestaltung, bei welcher das Katalysatorelement ganz oder abschnittsweise das Heizelement umschließt, ist denkbar. Weiterhin kann das optionale Katalysatorelement, wie oben ausgeführt, auch ganz oder teilweise als katalytische Beschichtung ausgestaltet sein oder eine katalytische Beschichtung umfassen, beispielsweise eine katalytische Beschichtung des Heizstabes und insbesondere der Glühkerze. Alternativ kann das Katalysatorelement als Hülse auf den Glühstift aufgebracht sein. Weiterhin kann das Katalysatorelement auf der Elektrode angebracht sein, insbesondere als Ringelektrode mit einem Spalt zu dem Heizelement.

Das optionale Katalysatorelement kann insbesondere beheizbar ausgestaltet sein. So kann das Katalysatorelement beispielsweise elektrisch beheizbar sein, beispielsweise über mindestens einen Heizwiderstand, welcher Bestandteil des Katalysatorelements sein kann und/oder anderweitig mit dem Katalysatorelement verbunden sein kann oder mit diesem zusammenwirken kann. Alternativ oder zusätzlich sind jedoch auch andere Ausgestaltungen einer Beheizung möglich, beispielsweise eine Infrarotheizung und/oder eine Heizung mittels Induktion. Insbesondere kann das Katalysatorelement jedoch ganz oder teilweise als katalytisch beschichtete Heizvorrichtung ausgestaltet sein, beispielsweise als katalytisch beschichtete elektrischer Heizvorrichtung, insbesondere als katalytisch beschichteter Heizwiderstand. Insbesondere kann die katalytisch beschichtete Heizvorrichtung mindestens ein Element aufweisen, ausgewählt aus der Gruppe bestehend aus: einer Heizwendel mit katalytisch beschichteter Oberfläche; einer Heizwendel mit katalytisch beschichtetem Gehäuse.

Wie oben ausgeführt, kann die Messzelle insbesondere mindestens einen Innenraum aufweisen, welcher für das Gas zugänglich ist. So kann beispielsweise die Messzelle mindestens ein Gehäuse mit mindestens einem Innenraum aufweisen, wobei das Gehäuse mindestens einen Einlass für das Gas und mindestens einen Auslass für das Gas aufweist. Dabei können ein oder mehrere Einlässe vorgesehen sein, beispielsweise mindestens ein Einlass für das eigentliche Gas, in welchem der Kohlenwasserstoff-Anteil zu bestimmen ist, sowie mindestens ein weiterer Einlass, beispielsweise für einen Trägergas und/oder Luft. Weiterhin können auch ein oder mehrere Auslässe vorgesehen sein. Der mindestens eine Einlass und der mindestens eine Auslass können beispielsweise an axial zueinander versetzten Stellen des Gehäuses vorgesehen sein, so dass das Heizelement, das optionale Katalysatorelement und die Elektrode zumindest teilweise zwischen dem Einlass und dem Auslass angeordnet sind. Beispielsweise können der Einlass und der Auslass an einander gegenüberliegenden Seiten des Gehäuses vorgesehen sein.

Insbesondere können das Heizelement, das optionale Katalysatorelement und die Elektrode in der Messzelle derart angeordnet sein, dass das Gas bei einer Durchströmung des Innenraums von dem mindestens einen Einlass zu dem mindestens einen Auslass mit dem mindestens einen Heizelement, dem mindestens einen optionalen Katalysatorelement und der mindestens einen Elektrode in Kontakt gelangt.

Das Heizelement kann insbesondere auf einer Achse der Messzelle angeordnet sein. Beispielsweise kann die Messzelle zumindest teilweise oder zumindest im Wesentlichen zylindrisch ausgestaltet sein, wobei die Achse beispielsweise eine Zylinderachse der Messzelle sein kann. Das optionale Katalysatorelement kann, wie oben ausgeführt, das Heizelement insbesondere zumindest abschnittsweise ringförmig umschließen. Zwischen dem Katalysatorelement und dem Heizelement kann ein Ringspalt ausgebildet sein, wobei der Ringspalt beispielsweise zylindrisch mit konstanter Dicke oder auch zumindest abschnittsweise konstanter Dicke ausgestaltet sein kann. Der Ringspalt kann auch in mehrere Segmente unterteilt sein, welche beispielsweise unterschiedliche Dicke aufweisen können. Die Messzelle und der Innenraum können derart ausgestaltet sein und der mindestens eine Einlass und der mindestens eine Auslass können derart angeordnet sein, dass das Gas bei der Durchströmung des Innenraums von dem Einlass zu dem Auslass durch den Ringspalt strömt, insbesondere axial.

Die Sensorvorrichtung kann neben der Messzelle weitere Bestandteile enthalten. So kann beispielsweise mindestens eine Versorgungsvorrichtung zur Versorgung der Messzelle mit dem mindestens einen Gas sowie optional mindestens einem Trägergas und/oder Luft vorgesehen sein. Beispielsweise kann diese Versorgungsrichtung mindestens eine Zuleitung und/oder mindestens eine Ableitung umfassen. Weiterhin kann optional mindestens eine Pumpe vorgesehen sein, welche beispielsweise mit dem Auslass und/oder mit dem Einlass verbunden sein kann, um eine Durchströmung des Innenraums zu bewirken. Weiterhin kann mindestens ein Ventil vorgesehen sein, beispielsweise in mindestens einer Zuleitung zu dem Einlass und/oder in mindestens einer Ableitung von dem Auslass. Weiterhin können eine oder mehrere Druckmessvorrichtungen vorgesehen sein.

Die Sensorvorrichtung kann weiterhin mindestens eine Steuerung umfassen. Unter einer Steuerung kann dabei allgemein eine Vorrichtung verstanden werden, welche mindestens eine Funktion der Sensorvorrichtung ansteuert und/oder mindestens eine Information aus der Sensorvorrichtung ausliest. Die Steuerung kann beispielsweise als zentrale Steuerung ausgestaltet sein oder auch als dezentrale Steuerung mit mehreren Komponenten. Die Steuerung kann insbesondere mindestens eine Datenverarbeitungsvorrichtung umfassen, beispielsweise mindestens eine Datenverarbeitungsvorrichtung ausgewählt aus der Gruppe bestehend aus: einem Prozessor; einem anwendungsspezifischen integrierten Schaltkreis (ASIC), einem Field-Programmable Gate Array (FPGA). Die Steuerung kann weiterhin mindestens eine Schnittstelle umfassen, beispielsweise mindestens eine elektronische Schnittstelle zum Austausch von Daten und/oder Befehlen mit mindestens einer weiteren Vorrichtung und/oder mindestens eine Benutzerschnittstelle. Die Steuerung kann weiterhin beispielsweise mindestens einen flüchtigen und/oder nicht flüchtigen Datenspeicher umfassen. Die Steuerung kann spezifisch für die Sensorvorrichtung vorgesehen sein oder kann auch anderweitig einsetzbar sein. Beispielsweise kann die Steuerung auch ganz oder teilweise zusammengefasst werden mit einer Steuerung einer Vorrichtung und/oder eines Systems, in welchem die Sensorvorrichtung eingesetzt wird, beispielsweise einer Motorsteuerung eines Kraftfahrzeugs.

Die Steuerung kann weiterhin mindestens eine Messvorrichtung umfassen und/oder mit mindestens einer Messvorrichtung verbunden sein, wobei die Messvorrichtung insbesondere mit der mindestens einen Elektrode verbunden sein kann, um direkt oder indirekt den Ionenstrom zu erfassen und/oder zu messen. Insbesondere kann die Steuerung eingerichtet sein, um direkt oder indirekt, beispielsweise über mindestens eine Messvorrichtung, mindestens ein Messsignal der Elektrode zu erfassen. Die Steuerung kann weiterhin eingerichtet sein, beispielsweise durch eine programmtechnische Einrichtung, um das mindestens eine Messsignal ganz oder teilweise auszuwerten, insbesondere um mindestens eine Information über den Kohlenwasserstoff-Anteil in dem mindestens einen Gas zu gewinnen. Insbesondere kann die Steuerung eingerichtet sein, um mittels des Messsignals den Kohlenwasserstoff-Anteil in dem Gas zu bestimmen.

Wie oben ausgeführt, kann die Steuerung insbesondere mindestens einen Prozessor umfassen, wobei die Optionen des ASIC und des FPGA mit von dem Begriff des Prozessors umfasst sein sollen. Der Prozessor kann insbesondere programmtechnisch eingerichtet sein, um den Kohlenwasserstoff-Anteil in dem Gas mittels mindestens eines Verfahrens zu bestimmen, ausgewählt aus der Gruppe bestehend aus: einem vorgegebenen Auswerte-Algorithmus, wobei das Messsignal als mindestens ein Eingangsinformation des Auswerte-Algorithmus verwendet wird und wobei mittels des Auswerte-Algorithmus mindestens eine Ausgangsinformation erzeugt wird, welche den Kohlenwasserstoff-Anteil in dem Gas charakterisiert; mindestens einer Korrelationsfunktion, insbesondere mindestens einer Kalibrierinformation, welche den mindestens einen Eingangssignal mindestens eine Ausgangsinformation zuordnet, welche den Kohlenwasserstoff-Anteil in dem Gas charakterisiert; einer Tabelle, insbesondere einer Kalibrationstabelle, welche einer Mehrzahl an möglichen Messsignalen entsprechende Ausgangsinformationen zuordnet, welche den Kohlenwasserstoff-Anteil in dem Gas charakterisieren. Insbesondere können durch mindestens eine Kalibrationsmessung oder Eichmessung der Auswerte-Algorithmus, die Korrelationsfunktion, die Kalibrierinformation, die Tabelle oder Kalibrationstabelle oder mindestens eine andere Art von Kalibrierinformation ermittelt werden, wie der Fachmann erkennen wird. So kann beispielsweise mindestens ein Kalibriergas mit bekanntem Kohlenwasserstoff-Anteil verwendet werden, das Messsignal erfasst werden und daraus mindestens eine Kalibrierinformation gewonnen werden. Auf diese Weise kann beispielsweise eine Korrelationsfunktion, beispielsweise eine Kalibrationsgerade oder Kalibrationskurve, welche einen Zusammenhang zwischen dem Kohlenwasserstoff-Anteil und dem Messsignal herstellt, generiert werden, oder auch eine Tabelle, in welcher für diskrete Messsignale entsprechende Kohlenwasserstoff-Anteile hinterlegt sind, beispielsweise eine Lookup-Table.

Korrelationen zwischen einer Kohlenwasserstoff-Konzentration und dem Ionenstrom können grundsätzlich von einigen Betriebsparametern abhängen. Die Betriebsparameter können in dem Auswerte-Algorithmus berücksichtigt werden. Die Betriebsparameter können mindestens einen Parameter umfassen ausgewählt aus der Gruppe bestehend aus: einem zu messenden Kohlenwasserstoff, insbesondere Methan und/oder Benzin und/oder Propan; einem Volumenstrom; einer Heizelementtemperatur des Heizelements; einem Gasdruck; einer Fertigungsstreuung von Heizelement und Ionenstromsensor. Auch andere Betriebsparameter sind grundsätzlich denkbar. Für eine Auswertung des Signals können die Betriebsparameter beispielsweise durch mathematische Funktionen und/oder durch Tabellen in Form von mehrdimensionalen Kennfeldern beschrieben werden. Bei den mathematischen Funktionen kann es sich beispielsweise um Polynom-Funktionen, die mit Methoden des "Design of Experiments" bestimmbar sind, handeln. Auch andere mathematische Funktionen sind grundsätzlich denkbar.

Die Temperatur des Heizelements kann eine wichtige Betriebsgröße sein, insbesondere da die Temperatur des Heizelements allgemein einen großen Einfluss auf die Ionisierung des Probengases haben kann. Insbesondere kann die Steuerung eingerichtet sein, das Heizelement auf eine Solltemperatur zu regeln, beispielsweise über den elektrischen Widerstand. Für unterschiedliche Gase und Messbereiche können unterschiedliche Solltemperaturen vorgegeben sein. Über eine Sensor-Kaskade mit mehreren Heizelementen und Ionenstrommessstellen können mehrere Probengaskomponenten detektiert werden. Hiermit kann beispielsweise ein Quereinfluss durch andere Gasbestandteile kompensiert werden.

Für eine Wärmeübertragung von dem Heizelement auf das Gas kann eine Verweilzeit des Gases in der Sensorvorrichtung, insbesondere in der Messzelle, eine wichtige Rolle auf eine Temperaturerhöhung und Ionisierung des Gases, insbesondere des Probengases, spielen. Die Sensorvorrichtung daher kann weiterhin eingerichtet sein, eine getaktete Gaszufuhr, insbesondere Probengaszufuhr mittels eines Gasventils, zu gewährleisten. Beispielsweise kann das Gasventil ein elektromagnetisch betätigbares Gasventil sein. Eine Verweilzeit des Gases in der Sensorvorrichtung, insbesondere in der Messzelle, kann variierbar sein durch mindestens einen Parameter ausgewählt aus der Gruppe bestehend aus: einer Taktfrequenz, einer Öffnungsdauer des Gasventils, einer Schließdauer des Gasventils; einem Verhältnis der Öffnungsdauer des Gasventils zu der Schließdauer des Gasventils.

Auch andere Parameter sind grundsätzlich denkbar. Dies kann insbesondere vorteilhaft sein für eine Unterstützung einer Ionisierung von schwer ionisierbaren Stoffen.

Durch die getaktete Betriebsweise kann das Gas eine Temperaturrampe durchlaufen, welche einen zeitlichen Verlauf der Ionisierung bzw. des Ionenstroms beeinflussen kann. Eine Auswertung des zeitlichen Verlaufs kann abhängig sein von einer Konzentration der Kohlenwasserstoffe, von einer chemischen Zusammensetzung der Kohlenwasserstoffe und anderen ionisierbaren Gaskomponenten. Folglich können über die Auswertung des zeitlichen Ionenstromverlaufs auch Quereinflüsse von den anderen Gaskomponenten, beispielsweise durch im Gas vorliegendes Kohlenmonoxid, analysiert und bei der Aufbereitung des Messsignals zur Korrektur des Messsignals verwendet werden. Somit kann eine Unempfindlichkeit gegenüber Quereinflüssen durch andere Gasbestandteile erzielt werden.

Abmessungen der Messzelle können in einem Zielkonflikt zwischen Wärmeübertragung zwischen heißer Oberfläche und dem Gas - hier sind grundsätzlich kleine Abmessungen quer zu einer Strömungsrichtung erwünscht - und großen Abmessungen für eine hohe elektrische Durchschlagfestigkeit zwischen den Elektroden der Ionenstrommessung - hier ist grundsätzlich ein großer Elektrodenabstand erwünscht um mit hohen Spannungen für die Ionisierung arbeiten zu können- stehen.

In einem weiteren Aspekt wird ein Verfahren zur Erfassung mindestens eines Kohlenwasserstoff-Anteils in mindestens einem Gas vorgeschlagen. Das Verfahren umfasst die nachfolgend beschriebenen Schritte. Die Verfahrensschritte können in der genannten Reihenfolge durchgeführt werden. Auch eine andere Reihenfolge ist jedoch grundsätzlich möglich. Weiterhin können zwei oder mehrere der Verfahrensschritte zeitlich überlappend oder auch gleichzeitig durchgeführt werden. Weiterhin können einer, mehrere oder alle der Verfahrensschritte einfach oder auch wiederholt durchgeführt werden. Das Verfahren kann darüber hinaus weitere Verfahrensschritte umfassen, welche nicht genannt sind.

Das Verfahren umfasst die folgenden Schritte:
a) Bereitstellen mindestens einer für das Gas zugänglichen Messzelle mit mindestens einem dem Gas zugänglichen Heizelement, sowie optional mindestens einem dem Gas zugänglichen Katalysatorelement zur Erzeugung von Ionen in dem Gas, weiterhin umfassend mindestens eine Elektrode, insbesondere ein Elektrodenpaar, zur Erfassung eines Ionenstroms in dem Gas;
b) Einbringen des Gases in die Messzelle;
c) Messung des Ionenstroms mittels der Elektrode; und
d) Bestimmung des Kohlenwasserstoff-Anteils mittels des gemessenen Ionenstroms.

Bezüglich möglicher Definitionen sowie möglicher Ausgestaltungen der in dem Verfahren genannten Elemente kann auf die obige Beschreibung der Sensorvorrichtung verwiesen werden. Insbesondere kann das Verfahren die Verwendung der vorgeschlagenen Sensorvorrichtung, beispielsweise gemäß einer der oben beschriebenen Ausgestaltungen oder auch gemäß einer der nachfolgend noch näher beschriebenen Ausgestaltungen, umfassen. Insbesondere kann das Heizelement zumindest teilweise als in die Messzelle hineinragender Heizstab ausgestaltet sein. Bei der Sensorvorrichtung wie auch dem Verfahren kann die mindestens eine Elektrode insbesondere mindestens ein Elektrodenpaar umfassen, da für eine Ionenstrommessung in der Regel zwei oder mehr Elektroden verwendet werden. Dabei kann die Rolle mindestens einer der Elektroden jedoch auch ganz oder teilweise von mindestens einem weiteren elektrisch leitfähigen Element übernommen werden, insbesondere die Rolle einer Massenelektrode. So kann beispielsweise eine elektrische leitfähige Gehäusewand mit einer elektrischen Masse verbunden sein und so als Masseelektrode dienen.

Wie oben bei der Beschreibung der Steuerung ausgeführt, kann das Verfahren weiterhin mindestens einen Kalibrierschritt umfassen. Dieser Kalibrierschritt kann die folgenden Teilschritte umfassen, wobei wiederum die Teilschritte in der genannten Reihenfolge durchgeführt werden können. Auch eine andere Reihenfolge ist jedoch grundsätzlich möglich. Weiterhin können zwei oder mehrere der Teilschritte zeitlich überlappend oder auch gleichzeitig durchgeführt werden. Weiterhin können einer, mehrere oder alle der Teilschritte einfach oder auch wiederholt durchgeführt werden. Der Kalibrierschritt kann darüber hinaus weitere Teilschritte umfassen, welche nicht genannt sind.

Der Kalibrierschritt umfasst die folgenden Teilschritte:
i. Einbringen mindestens eines Kalibriergases in die Messzelle, wobei das mindestens eine Kalibriergas mindestens einen vorgegebenen oder bekannten Kohlenwasserstoff-Anteil aufweist;
ii. Messung des Ionenstroms in dem Kalibriergas; und
iii. Bestimmung mindestens einer Kalibrierinformation mittels des gemessenen Ionenstroms und des Kohlenwasserstoff-Anteils in dem Kalibriergas.

Der Begriff "Kalibriergas", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein in dem Kalibrierschritt verwendetes Gas oder Gasgemisch beziehen, welches eine bekannte oder bestimmbare Zusammensetzung und insbesondere einen bekannten oder bestimmbaren Kohlenwasserstoff-Anteil aufweist.

Der Kalibrierschritt kann insbesondere getrennt von der Erfassung des Kohlenwasserstoff-Anteils in mindestens einem Gas mit den Verfahrensschritten a) bis d) erfolgen. Beispielsweise kann der Kalibrierschritt mindestens einmal vor Durchführung der eigentlichen Messung oder Erfassung des Kohlenwasserstoff-Anteils in dem mindestens einen Gas erfolgen. Alternativ oder zusätzlich kann der Kalibrierschritt auch anschließend erfolgen. Wiederum alternativ oder zusätzlich können die Verfahrensschritte a) bis d) zur Bestimmung des Kohlenwasserstoff-Anteils in dem Gas und der Kalibrierschritt auch derart erfolgen, dass der Kalibrierschritt beispielsweise in regelmäßigen oder unregelmäßigen Abständen wiederholt wird, um beispielsweise eine Kalibrierung der Sensorvorrichtung zu wiederholen oder zu bestätigen.

Die vorgeschlagene Sensorvorrichtung und das vorgeschlagene Verfahren weisen gegenüber bekannten Vorrichtungen und Verfahren der genannten Art eine Vielzahl von Vorteilen auf. So wird insgesamt eine robuste und auch strengen Sicherheitsanforderungen genügende Messung von Kohlenwasserstoffen in Gasen ermöglicht.

Beispielsweise wird eine Messung eines Kohlenwasserstoff-Anteils, insbesondere einer Konzentration an CₓH_{y}, beispielsweise in Abgasen von Verbrennungsmotoren, in Luft-Kraftstoffgemischen in Brennräumen, in Tankentlüftungssystemen möglich, eine Messung einer Methanemission auf Deponien oder ähnliche Anwendungen. Im Gegensatz zu vielen etablierten Messmethoden ist die vorgeschlagene Erfassung des Kohlenwasserstoff-Anteils auch für mobile Messungen geeignet. Die Messung der Kohlenwasserstoff-Konzentration kann insbesondere in einer Strömung des Gases, beispielsweise in einem Teilstrom von Abgasen, erfolgen. Die Erfassung des Kohlenwasserstoff-Anteils kann insbesondere an einer elektrisch regelbaren, heißen Oberfläche erfolgen, im Gegensatz zu dem bekannten Flammenionisationsverfahren, so dass auf eine Flamme verzichtet werden kann. Der mittels der vorgeschlagenen Sensorvorrichtung gemessene Ionenstrom kann insbesondere mit dem Kohlenwasserstoff-Anteil und insbesondere der Kohlenwasserstoff-Konzentration in dem Gas korrelieren.

Die Sensorvorrichtung lässt sich insbesondere als flammenloses System gestalten, wodurch ein einfacher Betrieb und eine einfache Steuerung möglich werden. Auf die Verwendung von Wasserstoff oder wasserstoffhaltigen Gasgemischen, beispielsweise als Trägergas oder als Gas für die Erzeugung der Flamme, kann verzichtet werden. Das Verfahren und die Sensorvorrichtungen können rein elektrisch durchgeführt werden, einschließlich der Ionisierung und der Messung des Ionenstroms, so dass eine leichte Regelbarkeit zu verzeichnen ist, insbesondere im Vergleich zur Flammenionisation. Das Verfahren und die Sensorvorrichtungen lassen sich mit niedrigem Energieverbrauch im Vergleich zu herkömmlichen Verfahren und Vorrichtungen betreiben. Da keine Flamme verwendet wird, ist zudem ein geringer Verschleiß zu verzeichnen, und ein Wartungsaufwand lässt sich, insbesondere im Vergleich zu herkömmlichen Flammenionisationsdetektoren, deutlich verringern. Weiterhin lassen sich geringere Investitionskosten verzeichnen. Zudem ist ein geringerer Aufwand an Betriebsstoffen erforderlich, und es ist eine höhere Zuverlässigkeit zu verzeichnen als bei den herkömmlichen Vorrichtungen und Verfahren.

Die Sensorvorrichtung und das Verfahren lassen sich auch mit effizienten Betriebsstrategien kombinieren. Weiterhin kann eine Messung beispielsweise im Kraftfahrzeug im Realbetrieb erfolgen, insbesondere im so genannten RDE. Insbesondere lassen sich das Verfahren und die Sensorvorrichtung auch in Erdgas-betriebenen Kraftfahrzeugen einsetzen, beispielsweise zur Messung eines Methanschlupfs. Auch in bereits vorhandene industrielle Prozesse sind das Verfahren und die Sensorvorrichtung leicht integrierbar, insbesondere da ein Bauraumbedarf bei der vorgeschlagenen Sensorvorrichtung und dem vorgeschlagenen Verfahren generell kleiner gestaltet werden kann als bei etablierten Methoden, insbesondere als bei der Flammenionisationsdetektion.

Insbesondere lässt sich die Sensorvorrichtung als ionenstrom-basierter, echtzeitfähiger Miniatur-Kohlenwasserstoff-Sensor gestalten, insbesondere für dynamische Messanforderungen in modernen Fahrzeugantrieben. Neben Sicherheitsanforderungen durch den bevorzugten Betrieb ohne Wasserstoff und einer hohen Genauigkeit lässt sich insbesondere eine funktionssichere Langzeitstabilität gewährleisten. Das Messprinzip des Miniatur-Sensors kann insbesondere auf der Messung einer von der chemischen Zusammensetzung des Gases und der Temperatur abhängigen elektrischen Leitfähigkeit des Gases basieren, welche mittels Ionenstrommesstechnik gemessen werden kann.

Die Messzelle kann insbesondere kompakt ausgestaltet werden und kann insbesondere strömungsoptimiert gestaltet werden. In der Messzelle können das Gas, auch als Messgas bezeichnet, und/oder die unverbrannten Kohlenwasserstoffe beispielsweise in Anwesenheit von Luftsauerstoff an mindestens einer beheizten, katalytischen Oberfläche des Heizelements und/oder des optionalen Katalysatorelements oxidiert oder auf andere Weise ionisiert werden. Die Sensorvorrichtung und das Verfahren können also insbesondere derart eingerichtet sein, dass der Messzelle Sauerstoff und/oder Luft und/oder ein anderes oxidierendes Gas zugeführt wird. Beispielsweise kann die Messzelle einen ersten Einlass für das Gas oder Messgases aufweisen, sowie einen zweiten Einlass für das oxidierende Gas, beispielsweise Luft und/oder Sauerstoff. Durch Anlegen einer Spannung an der mindestens einen Elektrode, beispielsweise an den Messelektroden, kann sich ein elektrisches Feld ausbilden, indem Ladungen der sich im Feld befindenden Ionen abfließen können. Die Oxidationsreaktion kann insbesondere exotherm verlaufen. Mit einer Wärmefreisetzung der exothermen Reaktion an dem optionalen Katalysatorelement, beispielsweise an der katalytisch beschichteten und beheizten Oberfläche des Katalysatorelements, kann der an der mindestens einen Elektrode aufgrund der Ionen fließende Strom zunehmen.

Der Strom an den Elektroden kann als Messsignal und/oder als Maß für die oxidierten Kohlenwasserstoffe verwendet werden. Dabei können eine oder mehrere Elektroden verwendet werden. Beispielsweise kann die Sensorvorrichtung mehrere Elektroden umfassen, welche auch in Strömungsrichtung des Gases versetzt zueinander angeordnet sein können, beispielsweise axial zueinander versetzt. Diese Elektroden können beispielsweise eine oder mehrere Referenzmessstellen in Strömungsrichtung bilden, mittels derer beispielsweise auch verschiedene Arten von Ionen separiert werden können. So kann beispielsweise eine Separierung von reaktionsfreudigen und eher reaktionsträgeren Kohlenwasserstoffen ermöglicht werden. So kann beispielsweise die Steuerung eingerichtet sein, um Ionenströme an mehreren in Strömungsrichtung zueinander versetzt angeordneten Elektroden, beispielsweise axial zueinander versetzt angeordneten Elektroden, zu erfassen. Die Steuerung kann beispielsweise aus diesen unterschiedlichen Ionenströmen die Anteile unterschiedlicher Arten von Kohlenwasserstoffen in dem Gas bestimmen.

Die Sensorvorrichtung kann beispielsweise mindestens eine geeignete Messschaltung oder Messanordnung umfassen, mittels derer die Ionenströme erfasst und optional verstärkt und/oder ganz oder teilweise aufbereitet werden können. Die Steuerung kann ganz oder teilweise als Auswerteeinheit ausgestaltet sein, um entsprechende Messwerte aufzunehmen und vollständig oder teilweise auszuwerten und in mindestens eine Information über den mindestens einen Kohlenwasserstoff-Anteil umzuwandeln.

Die Auswertung und/oder auch die optionale Kalibrierung kann mittels mindestens eines intelligenten Algorithmus erfolgen. Beispielsweise kann mindestens ein intelligenter Algorithmus auf Basis der Sensor-Fusion mit Serien-Sensorik wie beispielweise der Lambdasonde erfolgen. Die mindestens eine Sensorvorrichtung und/oder die mindestens eine Steuerung der mindestens einen Sensorvorrichtung können beispielsweise mindestens eine Schnittstelle aufweisen, beispielsweise eine Schnittstelle zu mindestens einem CAN-BUS. Ein Wirkzusammenhang relevanter Parameter wie beispielsweise der Temperatur des Heizstabes und/oder des optionalen Katalysatorelements, insbesondere der katalytisch beschichteten Oberfläche, das Luftverhältnis oder andere Parameter können beispielsweise zur Kalibrierung mittels einer geeigneten Versuchsplanung, insbesondere DoE (Design of Experiments), festgelegt werden und beispielsweise in die Bildung einer Kalibrierkonstanten einfließen.

Die vorgeschlagene Sensorvorrichtung weist weiterhin eine geringe thermische Trägheit auf. Insbesondere ist die thermische Trägheit geringer als im Falle alternativer Messverfahren zu verzeichnen. Das vorgeschlagene Verfahren lässt sich also mit hoher Messdynamik durchführen. Damit lassen sich also Echtzeit-Messmethoden realisieren, und beispielsweise auch ein Einsatz zur Emissionsmessung in der Realfahrt (RDE) ist möglich.

Als Kalibriergas lassen sich einfache, bekannte oder bestimmbare Gasgemische einsetzen. So lässt sich beispielsweise als Kalibriergase Mischungen aus Stickstoff und verschiedenen Anteilen eines Kohlenwasserstoff-Gases einsetzen, beispielsweise Propan. Beispielsweise kann als Kalibriergas eine Mischung von 4000 ppm Propan in Stickstoff verwendet werden. Als Referenzgas mit einem Kohlenwasserstoff-Anteil von Null, auch als Nullgas bezeichnet, lässt sich beispielsweise Umgebungsluft einsetzen, wobei in der Regel dann kein Ionenstrom messbar ist. Auf diese Weise lassen sich bereits mindestens zwei Messpunkte generieren, welche bereits ausreichend sind, um eine einfache Kalibriergerade und, als Steigung der Kalibriergeraden, eine Kalibrierkonstante zu bestimmen.

Im Gegensatz zum Flammenionisationsdetektor kann bei der vorgeschlagenen Sensorvorrichtung vollständig auf eine Flamme verzichtet werden, da eine Oxidation an dem Heizstab und/oder an dem optionalen Katalysatorelement erfolgt. Zur Beheizung kann insbesondere als Heizelement eine Glühkerze verwendet werden. Derartige Glühkerzen sind kommerziell verfügbar und kostengünstig. Beispielsweise lassen sich Glühkerzen einsetzen, wie Sie beispielsweise in Fino Scholl: "Study of Premixed Combustion Induced by Controlled Hot Surface Ignition in Stationary Gas Engines", Doctoral Thesis, Valladolid/Karlsruhe, 2017 (erhältlich unter http://www.mmt.hs-karlsruhe.de/downloads/IKKU/Doctoral_Thesis_Fino_Scholl_2017.pdf), S. 63-65 beschrieben werden. Auch andere Heizelemente sind jedoch grundsätzlich einsetzbar. Die Verwendung kommerzieller Glühkerzen bewirkt jedoch eine erhöhte Robustheit, da derartige Glühkerzen in der Regel bereits für Kraftfahrzeuganwendungen und damit für hohe Robustheitsanforderungen ausgelegt sind.

In DE 197 57 112 A1 wird ein Messverfahren beschrieben, welches einem Messprinzip einer Lambda-Sonde, insbesondere eines Sauerstoffsensors ähnelt. Es werden insbesondere sauerstoffionenleitende Festelektrolyten beschrieben. Im Gegensatz zu der vorliegenden Erfindung wird in DE 197 57 112 A1 der Ionenstrom durch eine diffusionsoffene Keramik, insbesondere durch eine für Sauerstoffatome und -ionen diffusionsoffene Keramik, realisiert. Bei der Sensorvorrichtung gemäß der vorliegenden Erfindung, wird der Ionenstrom direkt in dem Probengas ermittelt.

In CN 1 527 051 A wird ein Flammenionisationsdetektor beschrieben. Weiterhin wird in dieser Druckschrift ein Verfahren beschrieben, bei welchem der Temperaturanstieg für die Ionisierung durch eine katalytisch unterstützte exotherme Reaktion des Probengases erfolgt. Dieses Verfahren ist dadurch also nur anwendbar, wenn es sich bei dem Probengas um ein reaktionsfähiges Gemisch, wie beispielsweise um ein Gemisch von Sauerstoff und Kohlenwasserstoffen, handelt. Damit hängt also auch die Ionisierung von der Probengaszusammensetzung ab. Bei der Sensorvorrichtung gemäß der vorliegenden Erfindung handelt es sich um eine flammenlose Sensorvorrichtung. Weiterhin kann die Sensorvorrichtung gemäß der vorliegenden Erfindung eingerichtet sein, eine Beheizung des Gases, insbesondere des Probengases, für die Ionisierung rein über das Heizelement, insbesondere über einen elektrisch beheizten Glühstift, zu gewährleisten. Daher können auch Gase, insbesondere Probengemische, die keine exotherme Reaktion ermöglichen, messbar sein.

JP S61-265 562 A beschreibt einen Flammen-Ionisations-Detektor. Hierbei wird durch eine katalytische Beschichtung die Verbrennung unterstützt. Auch bei diesem Messverfahren ist ein brennfähiges Gemisch, beispielsweise ein wasserstoff- und sauerstoffhaltiges Gemisch, erforderlich. Bei der Sensorvorrichtung gemäß der vorliegenden Erfindung handelt es sich um eine flammenlose Sensorvorrichtung. Die Sensorvorrichtung gemäß der vorliegenden Erfindung kann insbesondere eingerichtet sein, ohne offene Flamme auch nicht brennfähige Gaszusammensetzungen zu ionisieren.

In DE 195 02 285 A1 wird ein Verfahren beschrieben, das durch Beheizung Ionen erzeugen kann. Eine Messung eines Ionenstromsignals in einem elektrischen Feld zwischen zwei Elektroden mit Spannungsdifferenz erfolgt jedoch nicht. Stattdessen erfolgt die Detektion der Elektroden über einen Detektor eines Gaschromatographen. Das Ausgabesignal ist dabei ein Spektrum. Im Gegensatz zu der Sensorvorrichtung gemäß der vorliegenden Erfindung ist bei dem Verfahren gemäß DE 195 02 285 A1 die Verwendung eines ReaktivGases erforderlich um eine exotherme Reaktion ausgelöst durch die hohe Temperatur der Beheizung zu ermöglichen. Bei der Sensorvorrichtung gemäß der vorliegenden Erfindung kann vollständig auf ein Reaktivgas verzichtet werden, da durch ausreichend hohe Temperaturen das Probengas direkt ionisiert wird.

### Kurze Beschreibung der Figuren

Weitere Einzelheiten und Merkmale ergeben sich aus der nachfolgenden Beschreibung von Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schema-tisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:
- Figuren 1A bis 1C: ein exemplarisches Ausführungsbeispiel einer erfindungsgemäßen Sensorvorrichtung in einer perspektivischen Darstellung (Figur 1A), einer Schnittdarstellung eines Ausschnitts (Figur 1B) sowie einer Schnittdarstellung eines Heizelements des exemplarischen Ausführungsbeispiels (Figur 1C);
- Figur 2: eine schematische Darstellung eines erfindungsgemäßen Verfahrens zur Erfassung mindestens eines Kohlenwasserstoff-Anteils in mindestens einem Gas; und
- Figuren 3A bis 3C: einen Zusammenhang zwischen einem elektrischen Widerstand R und einer Oberflächentemperatur T eines Heizelements einer erfindungsgemäßen Sensorvorrichtung (Figur 3A) und Aufheizverläufe des Heizelements in der Sensorvorrichtung (Figuren 3A und 3B).

### Beschreibung der Ausführungsbeispiele

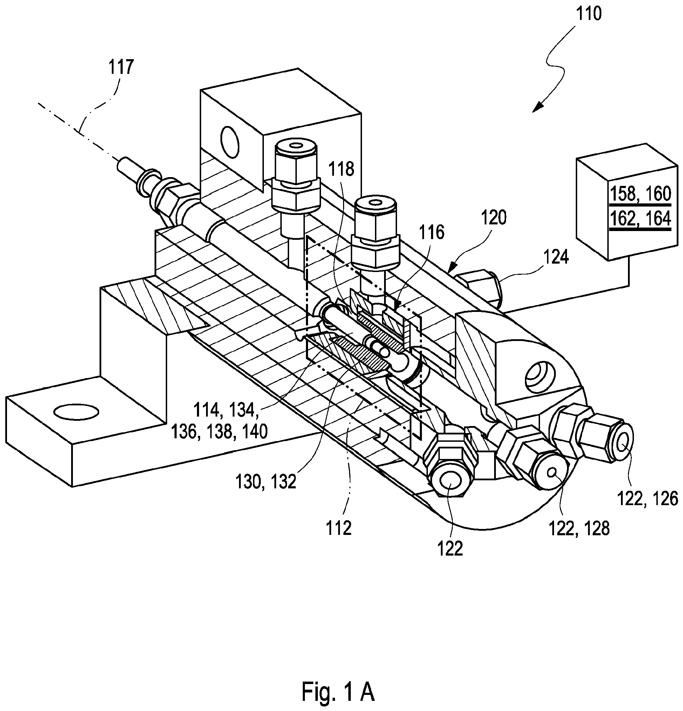

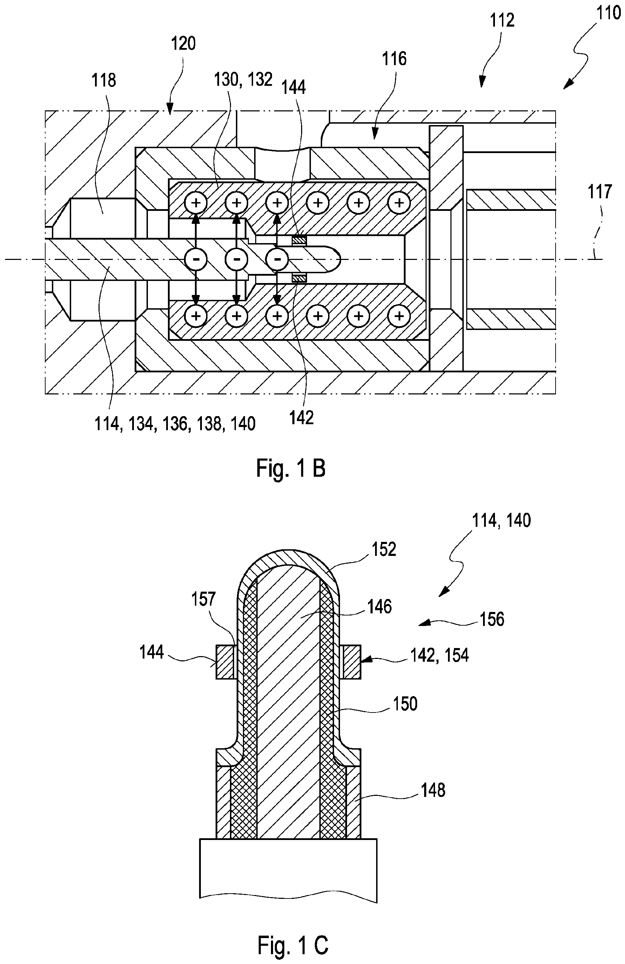

Figuren 1A bis 1C zeigen ein exemplarisches Ausführungsbeispiel einer erfindungsgemäßen Sensorvorrichtung 110 in einer perspektivischen Darstellung (Figur 1A), einer Schnittdarstellung eines Ausschnitts 112 (Figur 1B) sowie einer Schnittdarstellung eines Heizelements 114 des exemplarischen Ausführungsbeispiels (Figur 1C).

Die Sensorvorrichtung 110 weist, wie in Figur 1A dargestellt, eine für das Gas zugängliche Messzelle 116 mit dem Heizelement 114 auf. Beispielsweise kann die Messzelle 116 zumindest teilweise zylindrisch ausgestaltet sein. Die Messzelle 116 kann mindestens eine Achse 117 aufweisen, welche sich entlang einer Längsseite des Zylinders erstreckt. Die Messzelle 116 kann insbesondere mindestens einen Innenraum 118 aufweisen, welcher für das Gas zugänglich ist. So kann beispielsweise die Messzelle 116 mindestens ein Gehäuse 120 dem Innenraum 118 aufweisen. Das Gehäuse 120 kann mindestens einen Einlass 122 für das Gas und mindestens einen Auslass 124 für das Gas aufweisen. Dabei können einer oder mehrere Einlässe 122 vorgesehen sein, beispielsweise einen ersten Einlass 126 für das eigentliche Gas, in welchem der Kohlenwasserstoff-Anteil zu bestimmen ist, sowie ein zweiter Einlass 128, beispielsweise für einen Trägergas und/oder Luft.

Die Sensorvorrichtung 110 umfasst weiterhin mindestens eine Elektrode 130 zur Erfassung mindestens eines Ionenstroms in dem Gas. Beispielsweise können mindestens zwei gegensätzlich oder unterschiedlich geladene Elektroden 130 einen Bestandteil einer Wand 132 des Innenraums 118 bilden oder in anderer Weise für das Gas in dem Innenraum 118 zugänglich angeordnet sein. Die eine oder mehrere Elektroden 130 können beispielsweise über eine oder mehrere Zuleitungen (nicht dargestellt) mit einem Elektrodenpotenzial beaufschlagbar sein, so dass beispielsweise über die Zuleitung der Ionenstrom in dem Gas initiiert werden kann und auch als elektrischer Strom durch die Zuleitung gemessen werden kann.

Weiterhin umfasst die Sensorvorrichtung 110 das Heizelement 114. Insbesondere kann das Heizelement 114 mindestens ein elektrisches Heizelement 134 sein mit mindestens einem Heizwiderstand 136. Das Heizelement 114 kann zumindest teilweise als Heizstab 138 ausgestaltet sein. Insbesondere kann das Heizelement mindestens eine Glühkerze 140, welche nachfolgend in Figur 1C noch näher beschrieben wird.

Darüber hinaus kann die Sensorvorrichtung 110, wie in Figur 1B dargestellt, mindestens ein dem Gas zugängliches Katalysatorelement 142 zur Erzeugung von Ionen in dem Gas aufweisen. Das Katalysatorelement 142 kann mindestens eine Katalysatoroberfläche 144 aufweisen, welche direkt oder indirekt mit dem Gas in Verbindung gerät, so dass eine katalytische Reaktion durch das Katalysatorelement in dem Gas bewirkt oder begünstigt werden kann.

Das Katalysatorelement 142 kann insbesondere derart ausgestaltet sein, dass das Katalysatorelement 142 das Heizelement 114 zumindest teilweise ringförmig umschließt. Dies ist in Figur 1C schematisch dargestellt. Die Glühkerze 140 gemäß Figur 1C kann mindestens einen inneren Leiter 146 aufweisen. Ein äußerer Leiter 148 kann von dem inneren Leiter 146 durch mindestens einen Isolator 150 getrennt sein. Auf dem äußeren Leiter 148 kann eine Heizkappe 152 aufgesetzt sein. Das Katalysatorelement 142 kann dann beispielsweise ganz oder abschnittsweise als Hohlzylinder 154 ausgebildet sein und kann die Glühkerze 140 in mindestens einem Abschnitt 156 konzentrisch umschließen. Alternativ kann das Katalysatorelement 142 als Oberflächenbeschichtung oder als Hülse auf den Glühstift aufgebracht werden. Alternativ oder zusätzlich kann das Katalysatorelement 142 auf der Elektrode 130 angebracht sein, insbesondere als Ringelektrode mit einem Spalt zu dem Heizelement 114 (nicht dargestellt). Das Katalysatorelement 142 kann daher wahlweise mit dem Heizelement 114, wie in Figur 1B dargestellt, oder alternativ mt der Elektrode 130 verbunden sein (nicht dargestellt). Zwischen dem Katalysatorelement 142 und dem Heizelement 114 kann ein Ringspalt 157 ausgebildet sein, wobei das Gas bei der Durchströmung des Innenraums 118 von dem Einlass 122 zu dem Auslass 124 axial durch den Ringspalt 157 strömt.

Die Sensorvorrichtung 110 kann weiterhin mindestens eine Steuerung 158 umfassen. Dies ist in Figur 1A schematisch dargestellt. Die Steuerung kann eine Datenverarbeitungsvorrichtung 160, insbesondere einen Prozessor 162 aufweisen. Die Steuerung 110 kann weiterhin mindestens eine Messvorrichtung 164 umfassen, wobei die Messvorrichtung 164 insbesondere mit der mindestens einen Elektrode 130 verbunden sein kann, um direkt oder indirekt den Ionenstrom zu erfassen und/oder zu messen. Insbesondere kann die Steuerung eingerichtet sein, um direkt oder indirekt, beispielsweise über mindestens eine Messvorrichtung, mindestens ein Messsignal der Elektrode zu erfassen. Die Steuerung kann weiterhin eingerichtet sein, beispielsweise durch eine programmtechnische Einrichtung, um das mindestens eine Messsignal ganz oder teilweise auszuwerten, insbesondere um mindestens eine Information über den Kohlenwasserstoff-Anteil in dem mindestens einen Gas zu gewinnen.

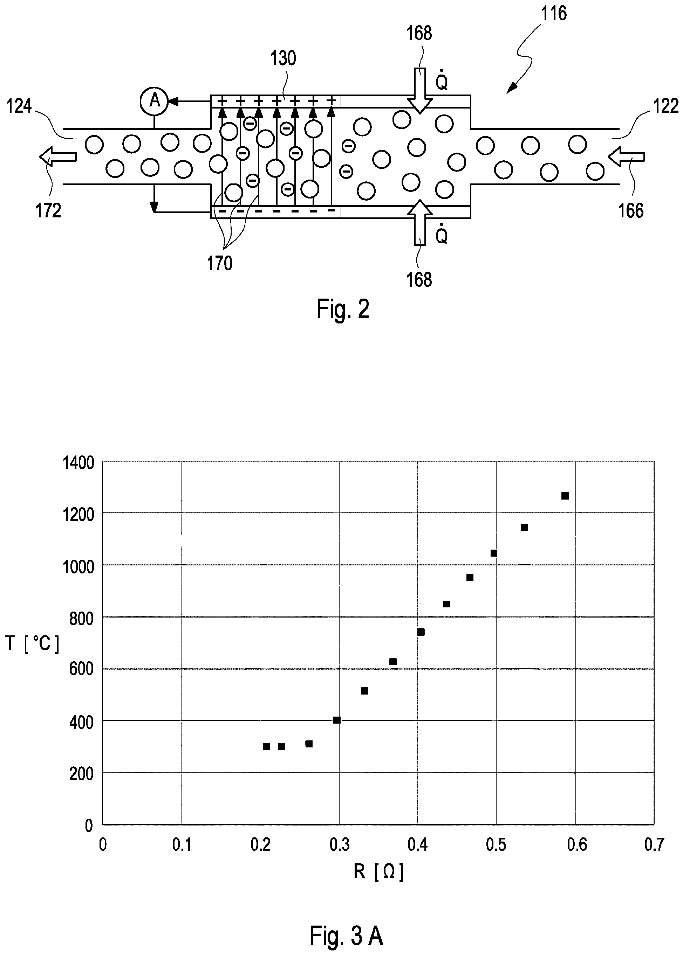

Figur 2 zeigt eine schematische Darstellung eines erfindungsgemäßen Verfahrens zur Erfassung mindestens eines Kohlenwasserstoff-Anteils in mindestens einem Gas.

Zunächst wird die für das Gas zugängliche Messzelle 116 mit dem Heizelement 114 (nicht dargestellt) und der Elektrode 130 bereitgestellt. In Figur 1 sind die Messzelle 116 sowie der Einlass 122 und der Auslass 124 sind dabei schematisch dargestellt. Anschließend kann das Gas in die Messzelle 116 über den Einlass 122 eingebracht werden, wie mit Pfeil 166 dargestellt. In einem nächsten Schritt kann das Gas durch einen Wärmeeintrag ionisiert werden, wie schematisch mit Pfeilen 168 dargestellt. Anschließend kann der Ionenstrom mittels der Elektrode 130 gemessen werden, wie schematisch mit Pfeilen 170 dargestellt. Der Kohlenwasserstoff-Anteil des Gases kann mittels des gemessenen Ionenstroms ermittelt werden. Da Gas kann aus dem Auslass 124 austreten, wie schematisch mit Pfeil 172 dargestellt.

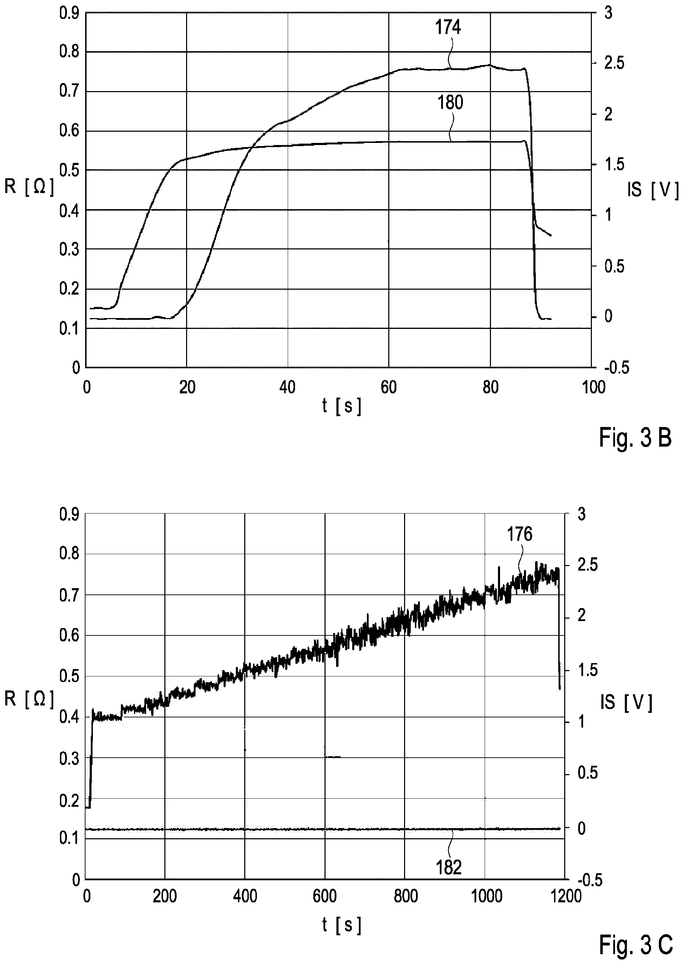

Figuren 3A bis 3C zeigen einen Zusammenhang zwischen einem elektrischen Widerstand R und einer Oberflächentemperatur T Heizelements 130 (Figur 3A) und Aufheizverläufe des Heizelements 130 in der Sensorvorrichtung 110 (Figuren 3A und 3B). Es wurde hierbei eine Sensorvorrichtung 110 gemäß der Figuren 1A bis 1C verwendet, sodass zumindest weitgehend auf die Beschreibung der Figuren 1A bis 1C oben verwiesen werden kann.

In Figur 3A ist ein Zusammenhang zwischen einem elektrischen Widerstand R und einer Oberflächentemperatur T Heizelements 130 dargestellt. Die Messung der Oberflächentemperatur T des Heizelements 130 erfolgte mittels eines Pyrometers in ruhendem Medium, nicht angeströmt.

In Figur 3B ist ein Aufheizverlauf des Heizelements 130 in der Sensorvorrichtung 110 dargestellt. Dabei wurde die Sensorvorrichtung 110 mit C₃H₈-haltigem Kalibriergas durchströmt. Es ist der elektrischen Widerstand R des Heizelements 130 in Abhängigkeit von einer Zeit t dargestellt. Es zeigte sich, dass ein Zusammenhang zwischen elektrischen Widerstand R des Heizelements 130, welcher mit der Oberflächentemperatur T des Heizelements 130 (siehe Figur 3A) korreliert, und der Messspannung des Ionenstroms besteht. Es ist eine Ionisierung erkennbar, insbesondere ab einem Widerstandswert von 0,5 Ohm. Für die Ionisierung wurde eine Gleichspannung zwischen den Elektroden von 120 V angelegt. In Figur 3B zeigt Linie 174 eine Messspannung des Ionenstroms. Die Messspannung der Auswerteeinheit ist proportional zum Ionenstrom. Linie 180 zeigt den elektrischen Widerstand des Heizelements 114. Der elektrische Widerstand korreliert mit der Temperatur des Heizelements 114, wie aus Fig 3A ersichtlich. Oberhalb des Widerstands von ca. 0,5 Ohm, das entspricht laut Fig. 3A einer Temperatur von ca. 1000 °C, ist bei Verwendung von HChaltigem Gas eine Ionisierung zu beobachten.

In Figur 3C ist ein Aufheizverlauf des Heizelements 130 in der Sensorvorrichtung 110 dargestellt. Dabei wurde die Sensorvorrichtung 110 mit kohlenwasserstofffreiem Nullgas durchströmt. Es ist der elektrischen Widerstand R in Abhängigkeit von einer Zeit t dargestellt. Es zeigte sich, dass ein Zusammenhang zwischen elektrischen Widerstand R des Heizelements 130, welcher mit der Oberflächentemperatur T des Heizelements 130 (siehe Figur 3A) korreliert, und der Messspannung des Ionenstroms besteht. Es ist keine Ionisierung erkennbar. Für die Ionisierung wurde eine Gleichspannung zwischen den Elektroden von 120 V angelegt. In Figur 3C zeigt Linie 176 einen elektrischen Widerstand des Heizelements 130. Der elektrische Widerstand korreliert mit der Temperatur des Heizelements 114, wie aus Fig 3A ersichtlich. Linie 182 zeigt die Messspannung des Ionenstroms. Die Messspannung der Auswerteeinheit ist proportional zum Ionenstrom. Es ergibt sich bei der Verwendung von kohlenwasserstofffreiem Nullgas keine Ionisierung.

### Bezugszeichenliste

- 110: Sensorvorrichtung
- 112: Ausschnitt
- 114: Heizelement
- 116: Messzelle
- 117: Achse
- 118: Innenraum
- 120: Gehäuse
- 122: Einlass
- 124: Auslass
- 126: erster Einlass
- 128: zweiter Einlass
- 130: Elektrode
- 132: Wand
- 134: elektrisches Heizelement
- 136: Heizwiderstand
- 138: Heizstab
- 140: Glühkerze
- 142: Katalysatorelement
- 144: Katalysatoroberfläche
- 146: innerer Leiter
- 148: äußerer Leiter
- 150: Isolator
- 152: Heizkappe
- 154: Hohlzylinder
- 156: Abschnitt
- 157: Ringspalt
- 158: Steuerung
- 160: Datenverarbeitungsvorrichtung
- 162: Prozessor
- 164: Messvorrichtung
- 166: Pfeil
- 168: Pfeil
- 170: Pfeil
- 172: Pfeil
- 174: Linie
- 176: Linie
- 180: Linie
- 182: Linie

## Patentansprüche

1. Sensorvorrichtung (110) zur Erfassung mindestens eines Kohlenwasserstoff-Anteils in mindestens einem Gas, umfassend eine für das Gas zugängliche Messzelle (116) mit mindestens einem dem Gas zugänglichen Heizelement (114), weiterhin umfassend mindestens eine Elektrode (130) zur Erfassung mindestens eines Ionenstroms in dem Gas.

2. Sensorvorrichtung (110) nach einem der vorhergehenden Ansprüche, wobei das Heizelement (114) zumindest teilweise als in die Messzelle (116) hineinragender Heizstab (138) ausgestaltet ist.

3. Sensorvorrichtung (110) nach einem der vorhergehenden Ansprüche, wobei das Heizelement (114) eine Glühkerze (140) umfasst.

4. Sensorvorrichtung (110) nach dem vorhergehenden Anspruch, weiterhin umfassend mindestens ein dem Gas zugängliches Katalysatorelement (142) zur Beeinflussung einer Ionenbildung in dem Gas.

5. Sensorvorrichtung (110) nach dem vorhergehenden Anspruch, wobei das Katalysatorelement (142) das Heizelement (114) zumindest teilweise ringförmig umschließt.

6. Sensorvorrichtung (110) nach einem der beiden vorhergehenden Ansprüche, wobei die Messzelle (116) mindestens ein Gehäuse (120) mit mindestens Innenraum (118) aufweist, wobei das Gehäuse (120) mindestens einen Einlass (122) für das Gas und mindestens einen Auslass (124) für das Gas aufweist, wobei das Heizelement (114), das Katalysatorelement (142) und die Elektrode (130) in der Messzelle (116) derart angeordnet sind, dass das Gas bei einer Durchströmung des Innenraums (118) von dem Einlass (122) zu dem Auslass (124) mit dem Heizelement (114), dem Katalysatorelement (142) und der Elektrode (130) in Kontakt gelangt.

7. Sensorvorrichtung (110) nach dem vorhergehenden Anspruch, wobei das Heizelement (114) auf einer Achse (117) der Messzelle (116) angeordnet ist, wobei das Katalysatorelement (142) das Heizelement (114) zumindest abschnittsweise ringförmig umschließt, wobei zwischen dem Katalysatorelement (142) und dem Heizelement (114) ein Ringspalt (157) ausgebildet ist, wobei das Gas bei der Durchströmung des Innenraums (118) von dem Einlass (122) zu dem Auslass (124) durch den Ringspalt (157) strömt.

8. Sensorvorrichtung (110) nach einem der vorhergehenden Ansprüche, weiterhin umfassend eine Steuerung (158), wobei die Steuerung (158) eingerichtet ist, um mindestens ein Messsignal der Elektrode (130) zu erfassen, wobei die Steuerung (158) weiterhin eingerichtet ist, um mittels des Messsignals den Kohlenwasserstoff-Anteil in dem Gas zu bestimmen.

9. Sensorvorrichtung (110) nach dem vorhergehenden Anspruch, wobei die Steuerung (158) mindestens einen Prozessor (162) umfasst, wobei der Prozessor (162) programmtechnisch eingerichtet ist, um den Kohlenwasserstoff-Anteil in dem Gas mittels mindestens eines Verfahrens zu bestimmen, ausgewählt aus der Gruppe bestehend aus: einem vorgegebenen Auswerte-Algorithmus, wobei das Messsignal als mindestens eine Eingangsinformation des Auswerte-Algorithmus verwendet wird und wobei mittels des Auswerte-Algorithmus mindestens eine Ausgangsinformation erzeugt wird, welche den Kohlenwasserstoff-Anteil in dem Gas charakterisiert; mindestens einer Korrelationsfunktion, insbesondere mindestens einer Kalibrierinformation, welche dem mindestens einen Eingangssignal mindestens eine Ausgangsinformation zuordnet, welche den Kohlenwasserstoff-Anteil in dem Gas charakterisiert; einer Tabelle, insbesondere einer Kalibrationstabelle, welche einer Mehrzahl an möglichen Messsignalen entsprechende Ausgangsinformationen zuordnet, welche den Kohlenwasserstoff-Anteil in dem Gas charakterisieren.

10. Sensorvorrichtung (110) nach einem der vorhergehenden Ansprüche, wobei die Sensorvorrichtung (110) eingerichtet ist, eine getaktete Gaszufuhr des Gases mittels eines Gasventils zu gewährleisten.

11. Sensorvorrichtung (110) nach dem vorhergehenden Anspruch, wobei eine Verweilzeit des Gases in der Sensorvorrichtung (110) variierbar ist durch mindestens einen Parameter ausgewählt aus der Gruppe bestehend aus: einer Taktfrequenz, einer Öffnungsdauer des Gasventils, einer Schließdauer des Gasventils, einem Verhältnis der Öffnungsdauer des Gasventils zu der Schließdauer des Gasventils.

12. Verfahren zur Erfassung mindestens eines Kohlenwasserstoff-Anteils in mindestens einem Gas, umfassend:
a) Bereitstellen mindestens einer für das Gas zugänglichen Messzelle (116) mit mindestens einem dem Gas zugänglichen Heizelement (114), weiterhin umfassend mindestens eine Elektrode (130) zur Erfassung eines Ionenstroms in dem Gas;
b) Einbringen des Gases in die Messzelle (116);
c) Messung des Ionenstroms mittels der Elektrode (130); und
d) Bestimmung des Kohlenwasserstoff-Anteils mittels des gemessenen Ionenstroms.

13. Verfahren nach einem der vorhergehenden Verfahrensansprüche, weiterhin umfassend mindestens einen Kalibrierschritt, umfassend folgende Schritte:
i. Einbringen mindestens eines Kalibriergases in die Messzelle (116), wobei das mindestens eine Kalibriergas mindestens einen vorgegebenen oder bekannten Kohlenwasserstoff-Anteil aufweist;
ii. Messung des Ionenstroms in dem Kalibriergas; und
iii. Bestimmung mindestens einer Kalibrierinformation mittels des gemessenen Ionenstroms und des Kohlenwasserstoff-Anteils in dem Kalibriergas.
